# EUROPEAN PATENT SPECIFICATION

(11) **EP 4 294 385 B1**
(45) Date of publication and mention of the grant of the patent: **17.06.2026**
(21) Application number: 22706786.5
(22) Date of filing: 15.02.2022
(51) Int. Cl.: A61K 31/365, A61K 31/00, A61K 31/352, A61K 31/407, A61K 31/4184, A61K 31/5415, A61K 31/7084, A61K 45/06, A61K 47/00, A61P 31/12, A61P 31/14, A61P 35/00, A61K 31/05, A61K 31/12, A61K 31/133, A61K 31/343, A61K 31/366, A61K 31/519, A61K 31/5377, A61K 31/6615

(54) **COMBINATION OF A SERCA2 INHIBITOR AND A STING ACTIVATOR FOR USE IN TREATING AND/OR PREVENTING CANCER**
KOMBINATION EINES SERCA2-INHIBITORS UND EINES STING-AKTIVATORS ZUR VERWENDUNG BEI DER BEHANDLUNG UND/ODER VORBEUGUNG VON KREBS
COMBINAISON D'UN INHIBITEUR DE SERCA2 ET D'UN ACTIVATEUR DE STING POUR UTILISATION DANS LE TRAITEMENT ET/OU LA PRÉVENTION DU CANCER

(30) Priority: 17.02.2021 EP 21305196
(43) Date of publication of application: 27.12.2023
(73) Proprietor: Centre National de la Recherche Scientifique, 75016 Paris (FR); Université de Montpellier, 34090 Montpellier (FR)
(72) Inventor: LAGUETTE, Nadine, 34830 CLAPIERS (FR); VILA, Isabelle Krystel, 30610 LOGRIAN FLORIAN (FR); STEER, Alizée Julie, 34070 MONTPELLIER (FR)
(74) Representative: Novagraaf Technologies
(86) International application number: PCT/EP2022/053655
(87) International publication number: WO 2022/175257

(56) References cited:
- WO-A1-2015/077354
- WO-A1-2021/113605
- US-A1- 2021 008 190
- ANONYMOUS: "Aduro Biotech Presents Preliminary Results Phase 1 Trials of STING agonist ADU-S100", HOLLANDBIO, 9 November 2018 (2018-11-09), pages 1 - 7, XP055850563, Retrieved from the Internet <URL:https://www.hollandbio.nl/nieuws/aduro-biotech-presents-preliminary-results-phase-1-trials-of-sting-agonist-adu-s100/> [retrieved on 20211012]
- FAN LU ET AL: "Novel role of Sarco/endoplasmic reticulum calcium ATPase 2 in development of colorectal cancer and its regulation by F36, a curcumin analog", BIOMEDICINE AND PHARMACOTHERAPY, ELSEVIER, FR, vol. 68, no. 8, 29 October 2014 (2014-10-29), pages 1141 - 1148, XP029586756, ISSN: 0753-3322, DOI: 10.1016/J.BIOPHA.2014.10.014
- DENMEADE SAMUEL R ET AL: "Prostate-specific antigen-activated thapsigargin prodrug as targeted therapy for prostate cancer", JOURNAL OF THE NATIONAL CANCER INSTITUTE, OXFORD UNIVERSITY PRESS, GB, vol. 95, no. 13, 2 July 2003 (2003-07-02), pages 990 - 1000, XP009133725, ISSN: 0027-8874, DOI: 10.1093/JNCI/95.13.990
- CHEMALY ELIE R ET AL: "SERCA control of cell death and survival", CELL CALCIUM, vol. 69, 2018, pages 46 - 61, XP085319354, ISSN: 0143-4160, DOI: 10.1016/J.CECA.2017.07.001
- LIU YIJUN ET AL: "STING, a promising target for small molecular immune modulator: A review", EUROPEAN JOURNAL OF MEDICINAL CHEMISTRY, ELSEVIER, AMSTERDAM, NL, vol. 211, 18 December 2020 (2020-12-18), XP086466253, ISSN: 0223-5234, [retrieved on 20201218], DOI: 10.1016/J.EJMECH.2020.113113
- ZHU QINGYUAN ET AL: "A synthetic STING agonist inhibits the replication of human parainfluenza virus 3 and rhinovirus 16 through distinct mechanisms", ANTIVIRAL RESEARCH, ELSEVIER BV, NL, vol. 183, 17 September 2020 (2020-09-17), XP086328449, ISSN: 0166-3542, [retrieved on 20200917], DOI: 10.1016/J.ANTIVIRAL.2020.104933
- JIANG MINLIN ET AL: "cGAS-STING, an important pathway in cancer immunotherapy", JOURNAL OF HEMATOLOGY AND ONCOLOGY, 22 June 2020 (2020-06-22), England, pages 81 - 81, XP055851090, Retrieved from the Internet <URL:https://www.ncbi.nlm.nih.gov/pmc/articles/PMC7310007/pdf/13045_2020_Article_916.pdf> [retrieved on 20211013], DOI: 10.1186/s13045-020-00916-z
- GENOVESE ILARIA ET AL: "Mitochondria as the decision makers for cancer cell fate: from signaling pathways to therapeutic strategies", CELL CALCIUM, ELSEVIER, AMSTERDAM, NL, vol. 92, 16 October 2020 (2020-10-16), XP086358298, ISSN: 0143-4160, [retrieved on 20201016], DOI: 10.1016/J.CECA.2020.102308

## Description

The invention is set out in the appended set of claims.

The present invention relates to products comprising a) at least one inhibitor of SERCA2 (Sarco/Endoplasmic Reticulum Ca²⁺-ATPase 2) selected from the group consisting of thapsigargin, Mipsagargin, JQ-FT, cyclopiazonic acid, artemisinin, CAD204520, Casearin J, CXL017, DBHQ and sHA 14-1, and b) at least one activator of STING selected from the group consisting of DMXAA, ADU-S100, amidobenzimidazole-based STING agonists, G10, ulevostinag, 3-Amino-bicyclo[3.2.1]octan-8-ol and E7766 ,
as combination products for a simultaneous, separate or sequential use in the treatment and/or prevention of a cancer.

Especially, the invention relates to the use of at least one SERCA2 inhibitor for treating cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766. The invention also relates to an inhibitor of SERCA2 for use in preventing and/or treating a cancer in a subject treated by at least one activator of STING .

The STimulator of INterferon Genes (STING) protein is an endoplasmic reticulum (ER) resident protein that plays a central role in innate immunity. Indeed, STING is an adaptor protein that orchestrates transcriptional activation of type I interferons and inflammatory cytokines in the presence of pathological nucleic acid species. STING activation relies on the detection of dsDNA, ssDNA, or RNA:DNA hybrids by the cyclic GMP-AMP synthetase (cGAS) pathogen recognition receptor. Association of cGAS with these nucleic acid species in the cytosol was found to lead to cGAS-dependent synthesis of cyclic GMP-AMP (cGAMP). Interaction of cGAMP with STING activates a pathway that finally leads to the transcription of inflammatory cytokines and type I interferons.

Recent research effort has aimed to identify means to modulate the cGAS-STING pathway. In particular STING-targeting immunotherapies aim to activate inflammatory responses, in immunosuppressed contexts. For example, Demaria *et al* (Demaria, O. et al. STING activation of tumor endothelial cells initiates spontaneous and therapeutic antitumor immunity. Proc. Natl. Acad. Sci. U. S. A. 112, 15408-15413 (2015)) have shown that activation of STING by intra-tumoral injection of cGAMP potentiates the anti-tumor CD8 T cell responses in mouse models of melanoma or colon cancer leading to control of tumor growth. Tang *et al* (Tang, C. H. A. et al. Agonist-mediated activation of STING induces apoptosis in malignant B cells. Cancer Res. 76, 2137-2152 (2016)) have presented evidence that activation and/or overexpression of STING can trigger apoptosis in tumor cells, inducing the release of tumor antigens and therefore promoting recognition by the immune system.

FAN LU ET AL: "Novel role of Sarco/endoplasmic reticulum calcium ATPase 2 in development of colorectal cancer and its regulation by F36, a curcumin analog", BIOMEDICINE AND PHARMACOTHERAPY, ELSEVIER, FR, vol. 68, no. 8, 29 October 2014 (2014-10-29), pages 1141-1148, investigates the role of SERCA2 in the development of colorectal cancer. In particular, this document describes that SERCA2 expression was positively correlated with metastasis levels in colorectal cancer patients. This document also discloses a compound F36 which inhibits SERCA2 signaling and is more active in inhibiting cell proliferation in colorectal cancer cells.

DENMEADE SAMUEL R ET AL: "Prostate-specific antigen-activated thapsigargin prodrug as targeted therapy for prostate cancer", JOURNAL OF THE NATIONAL CANCER INSTITUTE, OXFORD UNIVERSITY PRESS, GB, vol. 95, no. 13, 2 July 2003 (2003-07-02), pages 990-1000, is a study of the efficacy of an L12ADT pre-drug (thapsigargin pre-drug) for the treatment of prostate cancer.

CHEMAL Y ELIE RETAL: "SERCA control of cell death and survival", CELL CALCIUM, vol. 69, 2018, pages 46-61, aims to study the function and role of SERCA, i.e. its deregulation in the control of apoptosis in different cell types and its configuration in pathologies, with a view to its implication in therapy.

LIU YIJUN ET AL: "STING, a promising target for small molecular immune modulator: A review", EUROPEAN JOURNAL OF MEDICINAL CHEMISTRY, ELSEVIER, AMSTERDAM, NL, vol. 211, 18 December 2020, is a review and describes the structure of STING and its role of STING in various pathologies, i.e. infections, inflammations and cancers.

ZHU QINGYUAN ET AL: "A synthetic STING agonist inhibits the replication of human parainfluenza virus 3 and rhinovirus 16 through distinct mechanisms", ANTIVIRAL RESEARCH, ELSEVIER BV, NL, vol. 183, 17 September 2020 describes that activation of the STING signaling pathway may provide a new approach for treatments against Parainfluenza virus (PIV) and rhinovirus (HRV). This paper also describes the effect of the dimeric amidobenzimidaozole molecule (diABZI) as a STING activator on viral replication of different viruses (PIV3 and HRV16).

WO 2015/077354 describes STING activators for the treatment of cancers. In particular, this document describes activators comprising compounds of the following formulae: 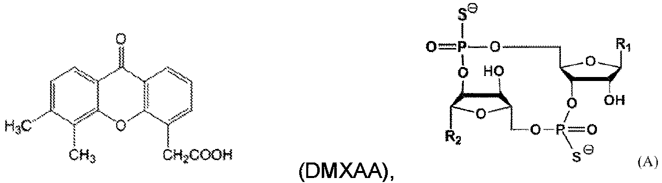 wherein R1 and R2 can be

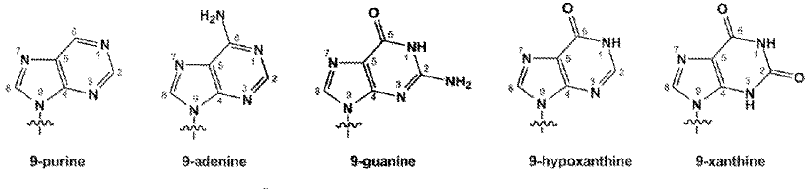

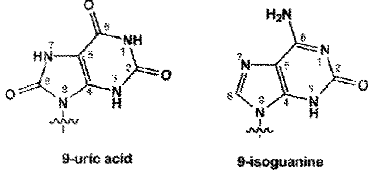

Jiang Minlin ET AL: "cGAS-STING, an important pathway in cancer immunotherapy",

Journal of hematology and oncology, 22 June 2020 (2020-06-22), pages 81-81, discloses that numerous STING activators have been developed for use in immunotherapeutics and mentioned that ADU-S100, MK-1454 activators have been approved in clinical trials for their ability to limit cancer progression.

GENOVESE ILARIA ET AL: "Mitochondria as the decision makers for cancer cell fate: from signaling pathways to therapeutic strategies", CELL CALCIUM, ELSEVIER, AMSTERDAM, NL, vol. 92, 16 October 2020 (2020-10-16) discloses the role of mitochondria in the inflammatory component of cancer, through the release of mitochondrial DNA involved in the activation of the cGAS-cGAMP-STING pathway and the uses of thapsigargin and mipsagargin.

US 2021/008190 discloses cell surface anchoring conjugates, formulations comprising cell surface anchoring conjugates, STING activators and methods of use thereof for enhancing a subject's immunity and treating tumor cells and cancer.

To date, all the molecules undergoing clinical trials for modulating the activation of STING directly act on this protein. The side effects, such as metabolic modulation associated with direct activation of STING (Vila IK et al. STING orchestrates the crosstalk between polyunsaturated fatty acid metabolism and inflammatory responses. Cell Metabolism. 34,125-139.e8 (2022)), and T lymphocyte apoptosis (Larkin B et al. Activation of STIGN in T cells induces type I IFN responses and cell death. J Immunol. 2017) are just starting to be characterized. Thus, there is a need for reducing these side effects and improving the efficacy of STING agonists. Especially, a subset of patients is refractory to conventional immunotherapies. There is thus a need for therapeutic agents that could overcome these therapeutic resistances.

To the contrary, several autoimmune or auto-inflammatory pathologies present with chronic STING activation. Such diseases include, but are not restricted to, Interferonopathies or STING-Associated Vasculopathy with onset in Infancy (SAVI). The former generally results from accumulation of pathological nucleic acids that activate STING, while the latter results from expression of constitutively active STING alleles. These pathologies are classically treated with inhibitors of the Janus kinase (JAK), blocking the type I IFN-signalling. Yet, these therapeutic approaches cause side effects, including increased susceptibility to pathogen infection, calling for alternative therapeutic strategies.

There is thus a need for novel and efficient therapies involving the STING pathway. There is also a need for novel and efficient drugs useful for preventing and/or treating cancer-related inflammation, while mitigating side effects.

The present invention solves these needs.

The references to methods of treatment in this description are to be interpreted as references to the compounds, pharmaceutical compositions and medicaments of the present invention for use in a method for treatment of the human (or animal) body by therapy.

Indeed, the inventors have surprisingly discovered that the Sarco/Endoplasmic Reticulum Ca²⁺-ATPase 2 (SERCA2) is a binding partner of STING. This new pathway is of major interest for treating pathologies involving STING activation, as well as for treating pathologies involving STING inhibition.

More specifically, the inventors sought to identify novel mechanisms involved in the regulation of STING. For this purpose, they immunopurified STING and protein partners, and used Mass Spectrometry analysis to reveal novel interactions. Amongst identified partners, they recovered SERCA2 as a binding partner of STING and this interaction was checked by Western Blot on immuno-precipitated Flag-tagged STING (data not shown). As shown in the example, the inventors then evidenced that the inhibition of SERCA2 increased the inflammatory response in wild-type cells, boosting the effect of a known STING agonist, as well as in a model of chronic STING activation.

In conclusion, they showed that SERCA2 is an inhibitor of STING, and SERCA2 inhibitors potentiate the interferon response both in chronic models of STING activation and upon acute stimulations.

Thus, modulating SERCA2 could improve the response to immunotherapies, while allowing the reduction of the doses of STING agonist to be administered.

As a consequence, the present description discloses the use of a SERCA2 modulator for treating an inflammation related pathology (not part of the claimed invention).

In a first embodiment, the present invention relates to the use of at least one SERCA2 inhibitor for preventing and/or treating cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

In the context of cancer, the inflammatory state of tumors is a determinant component of their outcome, and inflammation plays a crucial role at all stages of tumorigenesis, from the appearance of neoplasic lesions to metastatic spread. This inflammatory state also determines the immunogenicity of tumors and their response to immunotherapy, distinguishing "hot" tumors from "cold" tumors.

Activation of the cGAS and STING-based pro-inflammatory signaling pathway in tumor cells has been documented in recent years, and in specific cases, to inhibit tumor progression by a number of mechanisms, including the promotion of tumor recognition by the immune system, leading to their destruction. In addition, activation of the STING pathway has been reported to improve the response to immunotherapy. These aspects are not observed in all cancer cases; it depends in particular on the grade and the origin of the tumor.

To date, approaches to activate STING are under development. The inventors offer an alternative or combinatorial approach to activate STING by using at least one SERCA2 inhibitor.

Thus, in the context of a cancer for which an inflammatory response is required, the activation of STING and the interferon response may be induced by a SERCA2 inhibitor. Such a cancer may be one presenting a low immunogenicity, or a cancer where the response to immunotherapy and/or radiotherapy could be reinforced.

In the case of a viral infection, the interferon response is of major impact. By using an inhibitor of SERCA2, STING activation could be reinforced, leading to interferon response and to a faster elimination of the virus.

In the present description, the use of at least one SERCA2 activator to decrease inflammation associated with STING is also disclosed (not part of the claimed invention). This could be of interest for treating chronic infectious diseases that include chronic STING activation, as well as chronic cancer-related inflammation.

The present invention relates to products comprising:
a) at least one inhibitor of SERCA2 (Sarco/Endoplasmic Reticulum Ca²⁺-ATPase 2) selected from the group consisting of thapsigargin, Mipsagargin, JQ-FT, cyclopiazonic acid, artemisinin, CAD204520, Casearin J, CXL017, DBHQ and sHA 14-1, and
b) at least one activator of STING selected from the group consisting of DMXAA, ADU-S100, amidobenzimidazole-based STING agonists, G10, ulevostinag, 3-Amino-bicyclo[3.2.1]octan-8-ol and E7766 ,
as combination products for a simultaneous, separate or sequential use in the treatment and/or prevention of a cancer.

The present invention also relates to an inhibitor of SERCA2 for use in preventing and/or treating a cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017 DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

The present invention further relates to an inhibitor of SERCA2 for use in preventing and/or treating a cancer in a subject treated by at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1. and the activator of STING is selected from the group consisting of DMXAA, ADU-S100, amidobenzimidazole-based STING agonists, G10, ulevostinag, 3-Amino-bicyclo[3.2.1]octan-8-ol and E7766.

The invention will be better understood in view of the following.

SERCA2 (Sarco/Endoplasmic Reticulum Ca²⁺-ATPase 2), also called ATP2A2, is one of the 3 SERCA proteins that are responsible for creating a Ca²⁺ gradient from the cytosol to the lumen of the ER and as such is essential to the maintenance of Ca²⁺ homeostasis in cells. Interestingly, recent studies have documented that calcium (Ca²⁺) fluxes can influence the activation of STING. In absence of stimulation, the stromal interaction molecule 1 (STIM1) transmembrane calcium sensor of the ER interacts with STING, preventing its interaction with TBK1. In the presence of Ca²⁺, STIM1 promotes anchoring of STING in the ER membrane. When the Ca²⁺ of the ER is exhausted, STIM1 releases STING, allowing its translocation to the Golgi upon activation. Thus, Ca²⁺ fluxes are central to STING regulation. The amino acid sequence of human SERCA2 can be found in Uniprot under accession number P16615.

The present disclosure, not part of the invention, discloses of a SERCA2 modulator for treating an inflammation related-pathology.

By "modulator", it is meant a compound which is an inhibitor or an activator of SERCA2. The modulator is a ligand of SERCA2, i.e. it binds to SERCA2.

By "SERCA2 inhibitor" or "inhibitor of SERCA2", it is meant a compound that reversibly or irreversibly binds to SERCA2 and decreases its activity. A reversible binding is a noncovalent interaction between the inhibitor and SERCA2. An irreversible binding is a covalent interaction between the inhibitor and SERCA2. The inhibitor may be competitive or non-competitive, preferably the inhibitor is non-competitive. Preferably, the inhibitor is specific of SERCA2. By "specific" it is meant that the inhibitor has an IC50 *in vitro* for SERCA2 (for calcium entry) of less than 2.5 µM, preferably of less than 1 µM. Preferably, such an inhibitor is a small molecule.

According to the invention inhibitors of SERCA2 are is selected from the group consisting of thapsigargin, Mipsagargin, JQ-FT, cyclopiazonic acid (CPA), artemisinin, CAD204520, Casearin J, CXL017, DBHQ or sHA 14-1.

Examples of inhibitors of SERCA2 are notably already commercially available, as it is the case for thapsigargin or cyclopiazonic acid (CPA).

The inhibitor of SERCA2 used according the invention is selected from the group consisting of thapsigargin, Mipsagargin, JQ-FT, cyclopiazonic acid, artemisinin, CAD204520, Casearin J, CXL017, DBHQ or sHA 14-1.

Thapsigargin (IUPAC name: (3S,3aR,4S,6S,6aR,7S,8S,9bS)-6-(Acetyloxy)-4-(butyryloxy)-3,3a-dihydroxy-3,6,9-trimethyl-8-{[(2Z2)-2-methylbut-2-enoyl]oxy}-2-oxo-2,3,3a,4,5,6,6a,7,8,9b-decahydroazuleno[4,5-b]furan-7-yl octanoate) is the compound of formula (I) below:

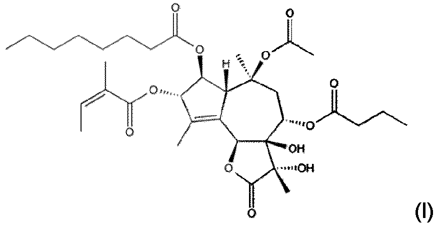

Thapsigargin is an irreversible SERCA2 inhibitor. It is a specific SERCA2 inhibitor, i.e. it shows an IC50 *in vitro* for SERCA2 (for calcium entry) of less than 15 nM.

CPA (IUPAC name: (6aR,11aS,11bR)-10-Acetyl-11-hydroxy-7,7-dimethyl-2,6,6a,7,11a,11b-hexahydro-9H-pyrrolo[1',2':2,3]isoindolo[4,5,6-cd]indol-9-one) is the compound of formula (II) below:

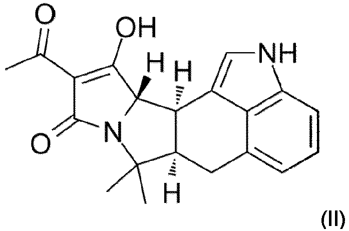

It is a specific SERCA2 inhibitor, i.e. it shows an IC50 *in vitro* for SERCA2 (for calcium entry) of less than 2500 nM.

Mipsagargin has the following structure:

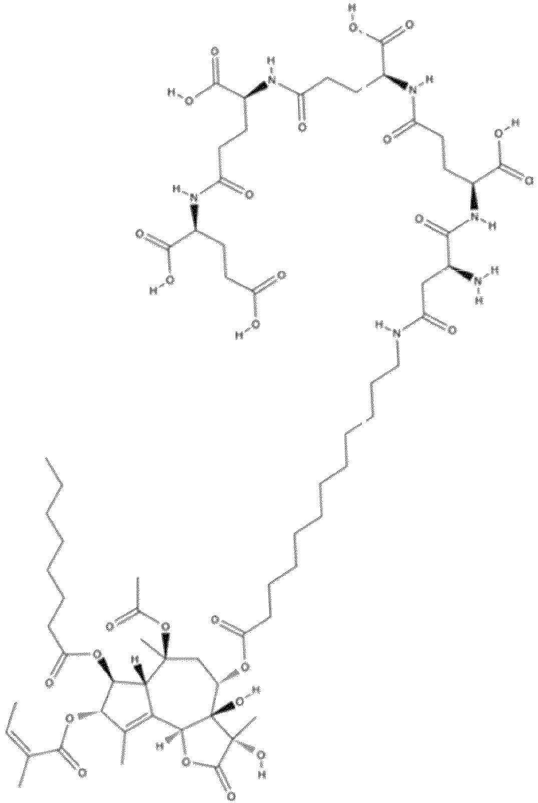

JQ-FT has the following structure:

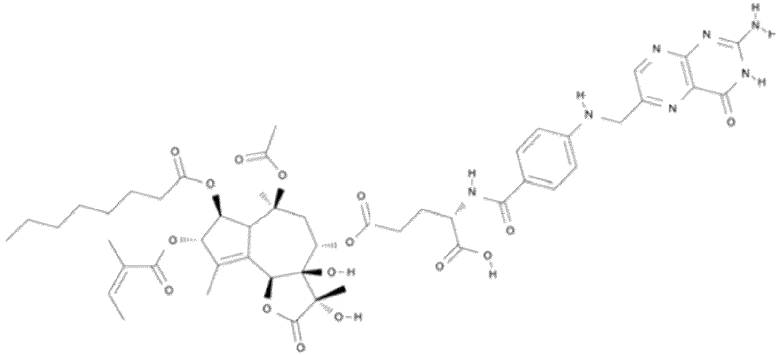

CAD204520 has the following structure:

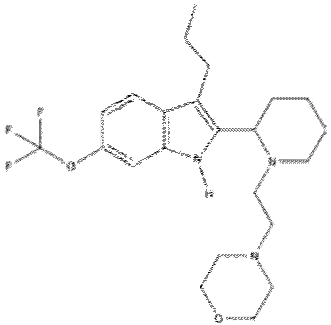

Casearin J has the following structure:

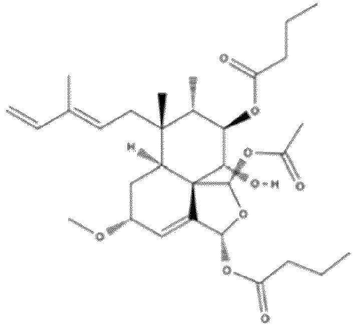

CXL017 has the following structure:

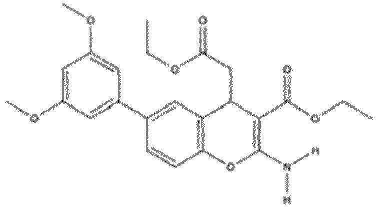

DBHQ has the following structure:

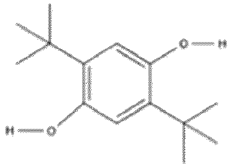

sHA 14-1 has the following structure:

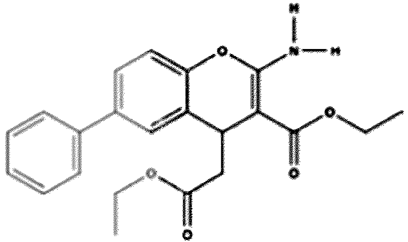

Artemisinin is a molecule of well-known structure.

By "SERCA2 activator" or "activator of SERCA2" (not part of the claimed invention), it is meant a compound that reversibly or irreversibly binds to SERCA2 and increases its activity. A reversible binding is a noncovalent interaction between the inhibitor and SERCA2. An irreversible binding is a covalent interaction between the activator and SERCA2. Preferably the activator is allosteric. Preferably the activator is a small molecule.

Examples of activators of SERCA2 are notably already commercially available, as it is the case for CDN-1163.

CDN-1163 (IUPAC name: N-(2-methylquinolin-8-yl)-4-propan-2-yloxybenzamide) is the compound of formula (III) below:

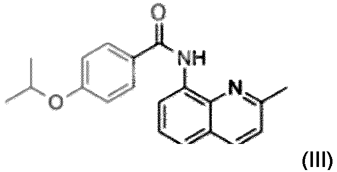

In order to determine whether a test compound is a SERCA2 inhibitor or a SERCA2 activator, the following protocol may be used for the measurement of calcium fluxes:
To measure the amount of calcium stored in endoplasmic reticulum after a test compound treatment, fluorescence will be measured in a medium lacking Ca2+ and Mg2+ in 96 well plates.

Cells will be loaded with Fluo-4 acetoxymethyl (Fluo-4 AM; Thermofisher) at 37°C for 30 minutes in the dark and then incubated with different concentrations of a test compound (for example 500 nM of thapsigargin) or control (DMSO). Fluo-4 AM is a labeled calcium indicator which is a molecule that exhibits an increase in fluorescence upon binding Ca²⁺.

Changes in fluorescence (485/530 nm) will be measured over a 5-minute period using a microplate reader. Data will be expressed as fluorescence units in test compound-treated cells minus the fluorescence units in the control cells.

If the fluorescence decreases with increasing concentrations of the test compound, then the test compound is a SERCA2 activator.

If the fluorescence increases with increasing concentrations of the test compound, then the test compound is a SERCA2 inhibitor.

The inhibitor of SERCA2 of the present invention is able to inhibit SERCA2 activity, thereby leading to STING activation, and to interferon response.

The use of said SERCA2 inhibitor in combination or in association with at least one activator of STING is for preventing and/or treating cancer.

An object of the invention is the use of a inhibitor of SERCA2 in preventing and/or treating a cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

Another object of the invention is the use of an inhibitor of SERCA2 for preventing and/or treating a cancer in a subject treated by at least one activator of STING, wherein the inhibitor of SERCA2 is selected from the group consisting of thapsigargin,

Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

The activator of SERCA2 of the present disclosure (not part of the claimed invention) is able to decrease inflammation associated with STING. The use of said activator is for treating chronic infectious diseases that include chronic STING activation, as well as cancer-related inflammation. Preferably, cancer-related inflammation involves the SERCA2-STING pathway.

By "inflammation", it is meant the inflammation that is triggered by activation of STING and that leads to type I interferon production (interferon response). Said inflammation is typically triggered in the presence of pathological immune-stimulatory nucleic acids that can either be endogenous (i.e. resulting from mitochondrial or nuclear damage for example) or exogenous (i.e. pathogen-derived). The inflammation may be acute or chronic. It includes cancer-related inflammation, but also the inflammation component of chronic inflammatory diseases such as lupus (i.e. systemic lupus erythematosus), Aicardi-Goutières syndrome, obesity, inflammatory bowel disease (IBD) or arthritis.

By "cancer-related inflammation", it is meant the inflammation which is due to cancer, or the inflammation which causes cancer. Said inflammation is characterized by the presence of pro-inflammatory cytokines (such as CxCL10 and ISG15) and of type I interferon. Particularly, the activator of SERCA2 of the disclosure is used for decreasing and/or inhibiting the production of type I interferon and/or pro-inflammatory cytokines (such as CxCL10 and ISG15) notably in cancer cells, via interaction with the SERCA2-STING pathway. Particularly, the inhibitor of SERCA2 of the invention is used for increasing and/or activating the production of type I interferon and/or pro-inflammatory cytokines (such as CxCL10 and ISG15), via interaction with the SERCA2-STING pathway.

By "treatment" of a given disease, it is meant the curative treatment of said disease. A curative treatment is defined as a treatment that completely treats (cures) or partially treats the disease.

In the case of a treatment of cancer, it is defined as a treatment that completely treats (cures) or partially treats cancer, i.e. induces tumor growth stabilization, retardation or regression.

The "subject" refers to any subject and typically designates a patient, preferably a subject afflicted by a DNA virus, such as Herpes Simplex Viruses, Varicella Zooster Virus; or a subject afflicted by a RNA virus, such as Retroviruses including lentiviruses such as Human Immunodeficiency Type I and II viruses; or a subject afflicted by an inflammatory disease, or undergoing a treatment of cancer such as immunotherapy, chemotherapy and/or radiotherapy. The subject may be afflicted by a cancer, or by a chronic inflammatory disease, notably chosen from lupus (i.e. systemic lupus erythematosus), Aicardi-Goutières syndrome, obesity, inflammatory bowel disease (IBD) and arthritis. The invention relates to the treatment or prevention of cancer. In any case, the subject is preferably a vertebrate, more preferably a mammal, even more preferably a human being. By "cancer", it is meant any type of cancer. The cancer may be solid or non-solid, and may be for example selected from a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia but also non solid cancers such as lymphoma.

Preferably, the cancer is a pancreatic cancer, a breast cancer, a prostate cancer, a lung cancer, a liver cancer, a bone cancer, a bladder cancer or a leukemia.

The modulator of the invention is preferably administered at a therapeutically effective amount or dose. As used herein, "a therapeutically effective amount or dose" refers to an amount of the modulator of the invention which prevents, removes, slows down the disease, or reduces or delays one or several symptoms or disorders caused by or associated with said disease in the subject, preferably a human being. The effective amount, and more generally the dosage regimen, of the modulator of the invention and pharmaceutical compositions thereof may be determined and adapted by the one skilled in the art. An effective dose can be determined by the use of conventional techniques and by observing results obtained under analogous circumstances. The therapeutically effective dose of the modulator of the invention will vary depending on the disease to be treated or prevented, its gravity, the route of administration, any co-therapy involved, the patient's age, weight, general medical condition, medical history, etc.

Typically, the amount of the modulator to be administered to a patient may range from about 0.01 to 500 mg/kg of body weight for a human patient. In a particular embodiment, the pharmaceutical composition according to the invention comprises 0.01 mg/kg to 300 mg/kg of the modulator of the invention, preferably from 0.01 mg/kg to 3 mg/kg, for instance from 25 to 300 mg/kg.

In a particular aspect, the modulator of the invention can be administered to the subject by intratumoral route, parenteral route, topical route, oral route or intravenous injection. The modulator of the invention may be administered to the subject daily (for example 1, 2, 3, 4, 5, 6 or 7 times a day) during several consecutive days, for example during 2 to 10 consecutive days, preferably from 3 to 6 consecutive days. Said treatment may be repeated during 1, 2, 3, 4, 5, 6 or 7 weeks, or every two or three weeks or every one, two or three months. Alternatively, several treatment cycles can be performed, optionally with a break period between two treatment cycles, for instance of 1, 2, 3, 4 or 5 weeks. The modulator of the invention can for example be administered as a single dose once a week, once every two weeks, or once a month. The treatment may be repeated one or several times per year. Doses are administered at appropriate intervals which can be determined by the skilled person. The amount chosen will depend on multiple factors, including the route of administration, duration of administration, time of administration, the elimination rate of the compound, or of the various products used in combination with said compound, the age, weight and physical condition of the patient and his/her medical history, and any other information known in medicine.

The administration route can be intratumoral, oral, topical or parenteral, typically rectal, sublingual, intranasal, intra-peritoneal (IP), intra-venous (IV), intra-arterial (IA), intramuscular (IM), intra-cerebellar, intrathecal, intratumoral and/or intradermal. The pharmaceutical composition is adapted for one or several of the above-mentioned routes. The pharmaceutical composition is preferably administered by intratumoral route. The pharmaceutical composition may be injected in the tumor (*in situ*) or near the tumor. Said intratumoral route preferably includes the administration of long lasting delivery forms.

The present description discloses a composition comprising, in a pharmaceutically acceptable medium, at least one SERCA2 modulator according to the invention. Such a composition comprises a pharmaceutically acceptable medium (or carrier).

The carrier must be "acceptable" in the sense of being compatible with the other ingredients of the formulations and not deleterious to the recipient thereof.

The pharmaceutical composition can be formulated as a long lasting delivery form. Such forms include polymeric nanoparticles and notably PLGA (Poly(lactic-co-glycolic acid)) nanoparticles, hydrogels or any other galenic form usable for intratumor delivery.

The pharmaceutical composition can be formulated as solutions in pharmaceutically compatible solvents or as gels, oils, emulsions, suspensions, or dispersions in suitable pharmaceutical solvents or vehicles, or as pills, tablets, capsules, powders, suppositories, etc. that contain solid vehicles in a way known in the art, possibly through dosage forms or devices providing sustained and/or delayed release. For this type of formulation, an agent such as cellulose, lipids, carbonates or starches are used advantageously.

Agents or vehicles that can be used in the formulations (liquid and/or injectable and/or solid) are excipients or inert vehicles, i.e. pharmaceutically inactive and non-toxic vehicles.

Mention may be made, for example, of saline, physiological, isotonic and/or buffered solutions, compatible with pharmaceutical use and known to those skilled in the art. The compositions may contain one or more agents or vehicles chosen from dispersants, solubilizers, stabilizers, preservatives, etc.

Particular examples are methylcellulose, hydroxymethylcellulose, carboxymethylcellulose, cyclodextrins, polysorbate 80, mannitol, gelatin, lactose, liposomes, vegetable oils or animal, acacia, etc. Preferably, vegetable oils are used.

Formulations suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations suitable for parenteral administration conveniently comprise a sterile oily or aqueous preparation of the active ingredient which is preferably isotonic with the blood of the recipient. Every such formulation can also contain other pharmaceutically compatible and non-toxic auxiliary agents, such as, e.g. stabilizers, antioxidants, binders, dyes, emulsifiers or flavoring substances.

The present invention relates to products comprising:
a) At least one inhibitor of SERCA2 (Sarco/Endoplasmic Reticulum Ca²⁺-ATPase 2) selected from the group consisting of thapsigargin, Mipsagargin, JQ-FT, cyclopiazonic acid, artemisinin, CAD204520, Casearin J, CXL017, DBHQ and sHA 14-1, and
b) at least one activator of STING selected from the group consisting of DMXAA, ADU-S100, amidobenzimidazole-based STING agonists, G10, ulevostinag, 3-Amino-bicyclo[3.2.1]octan-8-ol and E7766 ,
as combination products for a simultaneous, separate or sequential use in the treatment and/or prevention of cancer.

It also relates to the use of at least one inhibitor of SERCA2 according to the invention, for preventing and/or treating a cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

It further relates to the use of at least one inhibitor of SERCA2 according to the invention, for preventing and/or treating a cancer in a subject treated by at least one activator of STING wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

Inhibitor of SERCA2 is described above.

The activator of STING is generally a compound that reversibly or irreversibly binds to STING and increases its activity. Such an activator may be a small molecule. The activator of STING can be DMXAA; ADU-S100; ABZI (amidobenzimidazole)-based STING agonists (diABZls); G10; ulevostinag (also known as MK-1454) ; 3-Amino-bicyclo[3.2.1]octan-8-ol (also known as SB11285) ; E7766; MK-2118 ; GSK3745417 ; BMS-986301 ; BI-STING (also known as BI 1387446) ; TAK-676 ; TTI-10001 ; SNX 281 ; or SYNB1891.According to the invention , the activator of STING is selected from the group consisting of DMXAA; ADU-S100; ABZI (amidobenzimidazole)-based STING agonists (diABZls); G10; ulevostinag (also known as MK-1454) ; 3-Amino-bicyclo[3.2.1]octan-8-ol (also known as SB11285) ; or E7766.

Preferably, the activator of STING is DMXAA; ADU-S100; ABZI (amidobenzimidazole)-based STING agonists (diABZls) or G10.

The activator of STING used according the invention is selected from the group consisting of DMXAA, ADU-S100, amidobenzimidazole-based STING agonists, G10, ulevostinag, 3-Amino-bicyclo[3.2.1]octan-8-ol and E7766.

DMXAA, also called acid acetic-4-dimethylxanthenone-6,5, has the following structure :

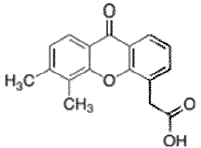

DMXAA is murine specific.

ADU-S100, also called MIW815, is a synthetic cyclic dinucleotide. It has the following structure :

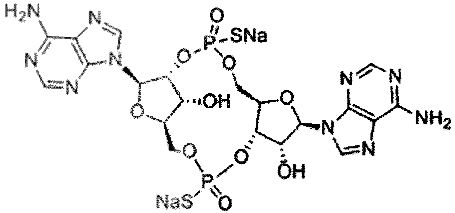

diABZls are STING agonists sharing ABZI core. A preferred diABZI compound is a potent non-nucleotide STING agonist of the following structure:

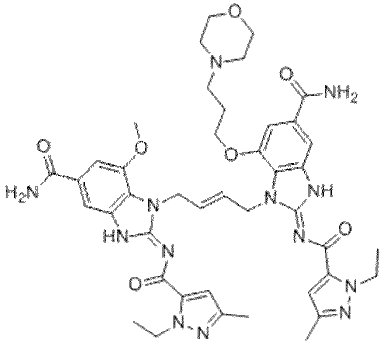

STING agonist-1 (G10) is a novel human-specific STING agonist. It has the following structure:

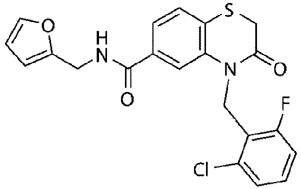

Ulevostinag (also known as MK-1454) is a STING agonist which has the following structure :

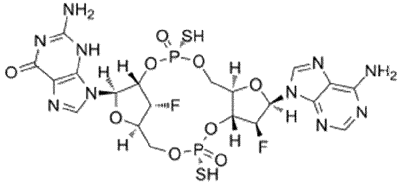

3-Amino-bicyclo[3.2.1]octan-8-ol (also known as SB11285) is a STING agonist which has the following structure:

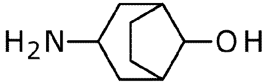

E7766 (CAS number 2242635-02-3) is a STING agonist which has the following structure:

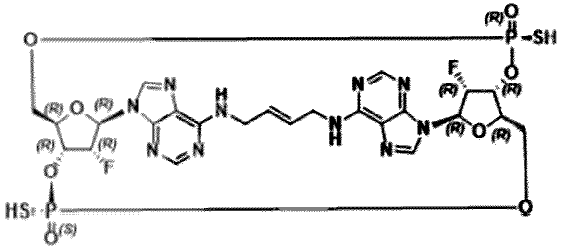

Finally, STING agonists may in general also be small molecules which are currently under clinical trials. Their structures are usually not disclosed. For example, MK-2118 is a STING agonist developed by Merck (small molecule), but its structure is undisclosed. GSK3745417 is a STING agonist developed by GSK (small molecule). BMS-986301 is a STING agonist developed by BMS (small molecule). BI-STING (also known as BI 1387446) is a STING agonist under clinical trials. TAK-676 is a STING agonist developed by Takeda. TTI-10001 is a STING agonist developed by Trillium Therapeutics Inc. SNX 281 is a STING agonist, being developed by Silicon Therapeutics. And SYNB1891 is a STING agonist developed by Synlogic.

The present invention is illustrated by the following figures:

### Figures

**Figure 1****: Inhibition of SERCA2 (ATP2A2) increased inflammatory response of WT-MEF cells.**
   WT-MEF cells were cultured 24h prior 2h stimulation with DMXAA (200µM) in combination or not with CPA (A-C) or thapsigargin (D-E). Graphs present fold increase Ifnβ, Cxcl10 and Isg15 mRNA levels, as compared to unstimulated cells. Graphs present mean value from duplicates.
**Figure 2****: Inhibition of SERCA2 (ATP2A2) increases inflammatory responses in Trex1 KO MEFs.**
   MEF^{Trexl-/-} cells were treated or not with CPA (A-C) or thapisgargin (D-E) for 2h. Graphs present fold increase Ifnβ, Cxcl10 and Isg15 mRNA levels, as compared to unstimulated cells. Graphs present mean value from duplicates (A-C) or average of 2 independent experiments (D-F). Error bars: standard deviation.

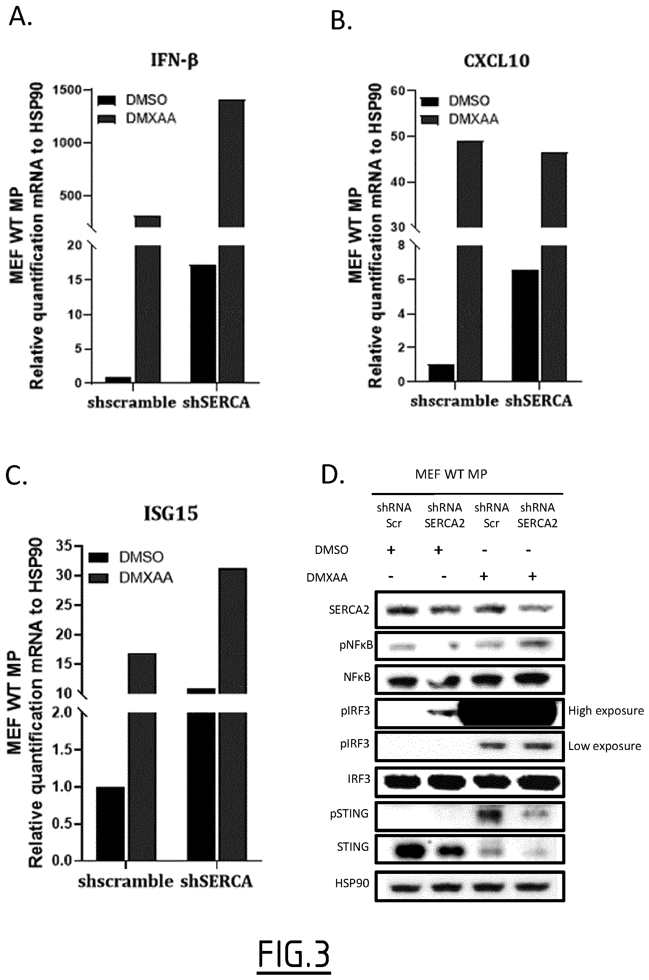

**Figure 3****: Knockdown of SERCA2 (ATP2A2) increases inflammatory responses in WT-MEFs.**
   MEF expressing non-targeting (Scramble) or ATP2A2-targeting shRNAs were stimulated or not with DMXAA (200µM) for 2h. (A-C) Graphs present mean fold change Ifnβ, Cxcl10 and Isg15 mRNA levels as measured by RT-qPCR. (D) WB analysis of whole-cell extracts of cells treated as in (A-C) using indicated antibodies.

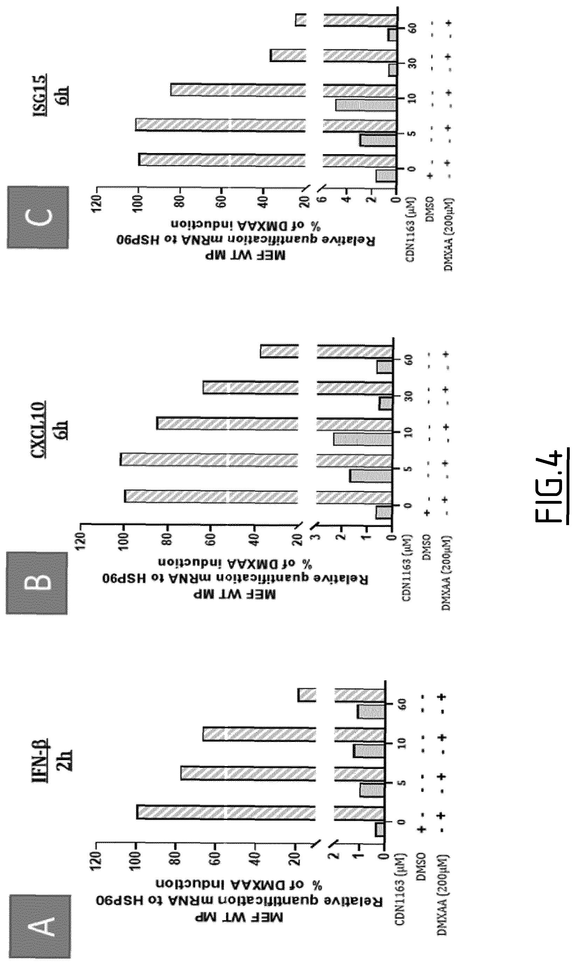

**Figure 4****: Treatment with the CDN-1163 SERCA2 (ATP2A2) allosteric activator decreases inflammatory responses in WT-MEFs.**
   WT-MEF cells were cultured 24h prior 2h stimulation with DMXAA (200µM) in combination or not with CDN-1163 (A-C). Graphs present fold increase Ifnβ, Cxcl10 and Isg15 mRNA levels, as compared to unstimulated cells. Graphs present mean value from duplicates.

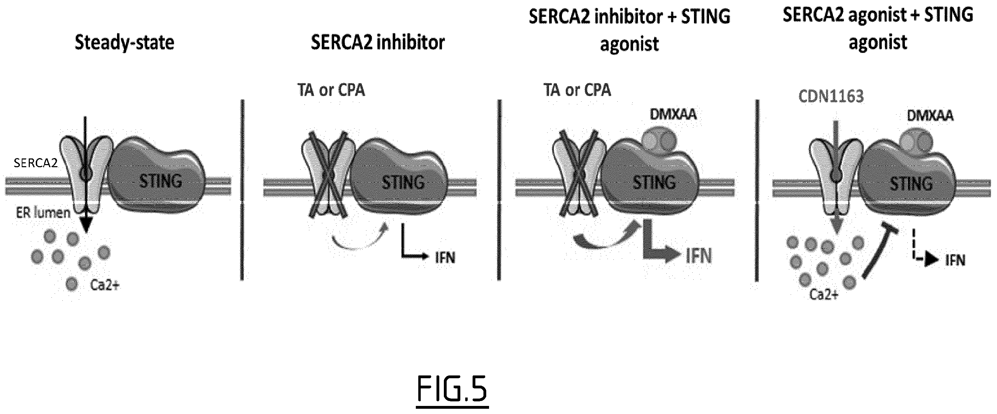

**Figure 5****: Impact of SERCA activity on the interferon response potentiation or inhibition.**

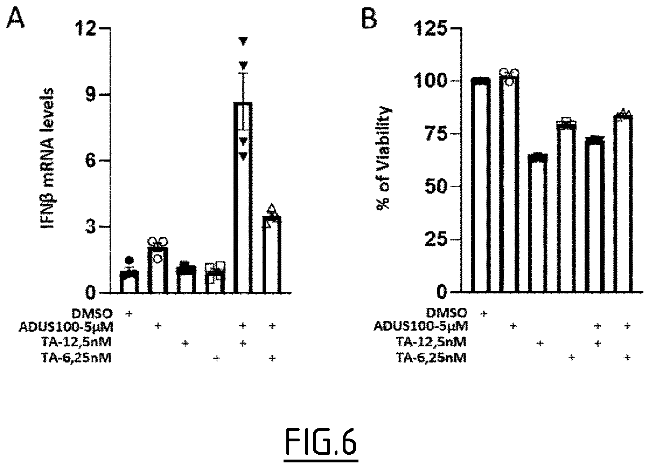

**Figure 6****: Inhibition of SERCA2 (ATP2A2) in combination with STING agonist increases inflammatory responses in mouse embryonic fibroblasts (MEFs).**
   (A) MEFs were cultured for 24h prior to 6h stimulation with ADUS-100 (5µM) in combination or not with 12.5nM or 6.25nM of thapsigargin (TA). Graphs present fold increase *Ifnβ* mRNA levels, as compared to unstimulated cells.
   (B) MEFs were cultured for 24h prior to 6h stimulation with ADUS-100 (5µM) in combination or not with 12.5nM or 6.25nM of TA. Graphs present viability level, as compared to unstimulated cells.
   Graphs present mean value from triplicates or quadriplicates.

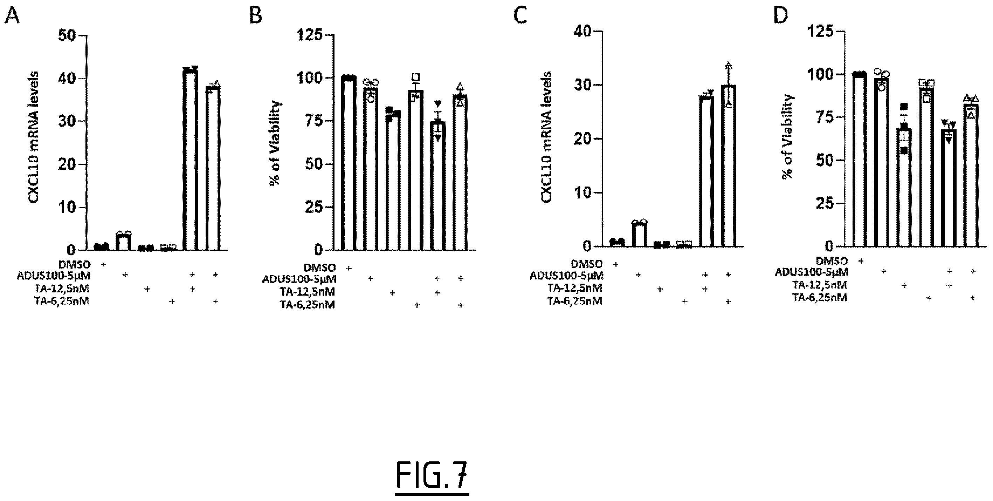

**Figure 7****: Inhibition of SERCA2 (ATP2A2) in combination of STING agonist increases inflammatory responses in cancer cell lines.**
   (A) The KPC murine pancreatic cell line was cultured 24h prior to 6h stimulation with ADUS-100 (5µM) in combination or not with 12.5nM or 6.25nM TA. Graphs present fold increase *Cxcl10* mRNA levels, an interferon responding gene, as compared to unstimulated cells.
   (B) KPC cells were cultured 24h prior 6h stimulation with ADUS-100 (5µM) in combination or not with 12.5nM or 6.25nM TA. Graphs present viability level, as compared to unstimulated cells.
   (C) The Panco2 murine pancreatic cell line was cultured 24h prior to 6h stimulation with ADUS-100 (5µM) in combination or not with 12.5nM or 6.25nM TA. Graphs present fold increase *Cxcl10* mRNA levels, an interferon responding gene, as compared to unstimulated cells.
   (D) Panco2 cells were cultured 24h prior to 6h stimulation with ADUS-100 (5µM) in combination or not with 12.5nM or 6.25nM TA. Graphs present viability level, as compared to unstimulated cells.
   Graphs present mean value from at least duplicates.

### Example 1: Modulation of SERCA2-dependent Calcium fluxes impacts the activation of STING-associated interferon responses

### MATERIALS AND METHODS:

### Cells and cell cultures

Wild type murine embryonic fibroblasts (WT-MEF) and MEF-Trex1^{-/-}, were maintained in Dulbecco's modified Eagle's medium (DMEM) supplemented with 10% fetal bovine serum (FBS), 1% penicillin/streptomycin and 1% glutamine.

WT-MEF were a gift of S. R. Paludan, MEFTrex1^{-/-} were obtained from J. Rehwinkel.

### Plasmids

shRNAs targeting ATP2A2 were obtained from Sigma-Aldrich (Clone ID:NM_009722.1-2015s1c1). shRNA-expressing lentiviral particles were produced by co-transfection of 2 × 10⁶ 293T cells with 5 µg of shSERCA2, 5 µg of psPAX2 GagPol, and 1 µg of p-MD2G envelope, using the standard calcium phosphate transfection protocol. Viral particles were harvested 24 hours after transfection, filtered with 0.45 µM filters, and used for transduction. For knockdown of SERCA2, 10⁶ MEF cells were seeded 24 hours before transduction. Medium was replaced 10 hours after transduction, and 1.5µg/ml puromycin selection was performed 72 hours later.

### RNA extraction and RT-qPCR

RNA was extracted using Trizol (Invitrogen) and treated with TURBO DNase (Ambion) according to manufacturer's protocols. Reverse transcription (SuperScript IV reverse transcriptase, Invitrogen) and qPCR using specific primers were performed using SYBR Green Master Mix (Takara) and LightCycler 480 cycler (Roche). mRNA levels were normalized to HSP90 mRNA levels.

### Whole-cell extract preparation and immunoblot

Cells were lysed in 30µl of TENTG-150 for 30 min at 4°C. Lysates were centrifuged 20 min at 12,000 rpm, and supernatants were collected for immunoblot. Protein quantification was performed using Bradford assay. Samples were resolved on SDS-PAGE, and proteins were transferred onto nitrocellulose membranes. Primary antibodies used include anti-phospho IRF3 (1:1000; Cell Signaling 4D4G), anti-IRF3 (1:1000; Cell Signaling D6I4C), anti-phospho STING S365 (1:1000; Cell Signaling D8F4W); anti-STING (1:1000; Cell Signaling D2P2F), anti-ATP2a2 (SERCA2) (1:1000, Cell Signaling D51B11); anti-HSP90 (1:1000; Cell Signaling C45G5); anti-phospho NFκβ p65 S536 (1:1000, Cell Signaling 93H1) and anti-NFκβ p65 (1:1000, Cell Signaling D14E12). All secondary antibodies (Cell Signaling) were used at 1:2000 dilution. Signal was visualized with SuperSignal West Pico Chemiluminescent Substrate (Thermo Fisher Scientific), and images were acquired on a ChemiDoc (Bio-Rad).

### Compounds (PubChem CID)

DMXAA (Vadimezan): 123964
CPA (Cyclopiazonic acid): 65261
Thapsigargin: 446378
CDN-1163: 16016585

### Oligonucleotide sequences

shATP2A2: 5'-CCGGCCAGGATTGAAGTAGCCTCTTCTCGAGAAGAGGCTACTTCAAT
CCTGGTTTTT-3' (SEQ ID NO:1).

mIFNβ fwd 5'-CTGCGTTCCTGCTGTGCTTCTCCA-3' (SEQ ID NO:2)
mIFNβ rev 5'-TTCTCCGTCATCTCCATA GGGATC-3' (SEQ ID NO:3)
mCXCL10 fwd 5'-ATGACGGGCCAGTGAGAATG-3' (SEQ ID NO:4)
mCXCL10 rev 5'-TGAACACGTGGGCAGGATAG-3' (SEQ ID NO:5)
mISG15 fwd 5'-GTGCTCCAGGACGGTCTTAC-3' (SEQ ID NO:6)
mISG15 rev 5'-CTCGCTGCAGTTCTGTACCA-3' (SEQ ID NO:7)

### RESULTS:

### Inhibition of SERCA2 (ATP2A2) increased inflammatory response of WT-MEF and in chronic inflammation model Trex I KO MEF

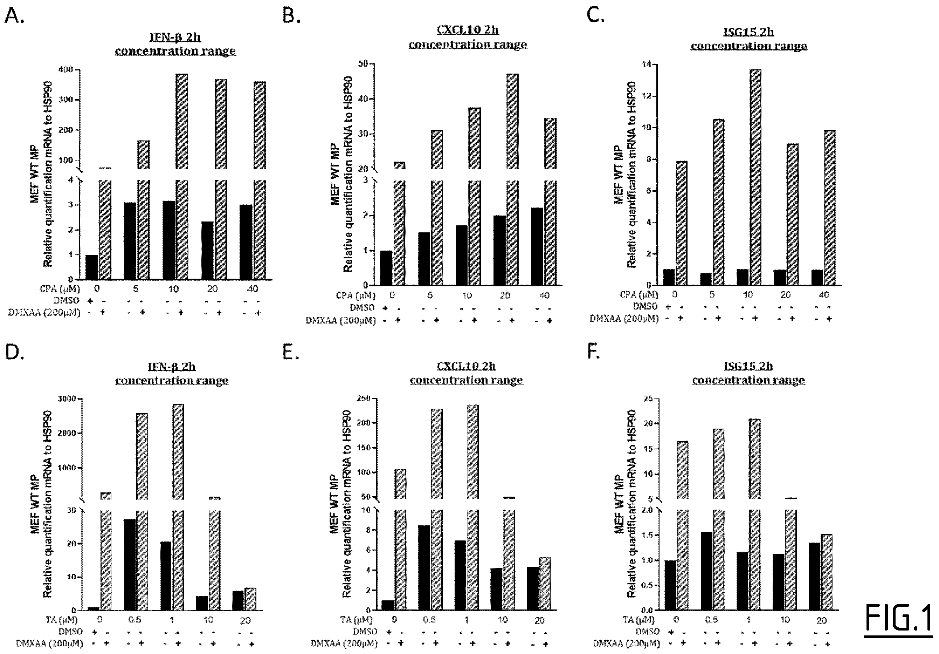

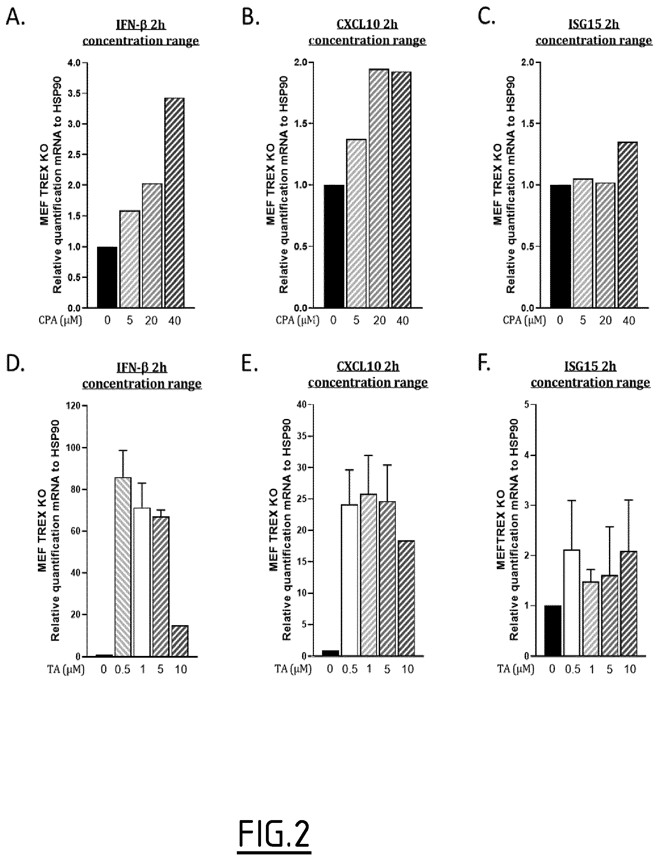

In order to investigate the impact of SERCA2 inhibition on inflammation associated with STING, two cellular models were used:
WT-MEF stimulated or not with DMXAA, a murine STING ligand which triggers STING-dependent inflammatory responses (Figure 1); and Trex I KO MEF which is a model of chronic STING activation (Figure 2).

Of note, Trex I deficiency is a model of type I interferonopathy.

WT-MEF or Trex I KO MEF were treated for 2h with SERCA2 inhibitors thapsigargin (TA) or CPA.

The inventors observed that treatment with SERCA2 inhibitors led to a potentiation of DMXAA-induced inflammation markers CXCL10 cytokine, Interferon Stimulated Gene (ISG-15) and Interferon-β (IFN-β) (Figure 1). Similarly, the inventors observed as well an increase of IFN-β, CXCL10 and ISG15 mRNA levels after CPA and TA treatment in Trex1-deficient cells (Figure 2).

Furthermore, the inventors confirmed that knocking-down SERCA2 before stimulation with DMXAA potentiates the expression of pro-inflammatory genes (Figure 3 A-C). In addition, they observed that SERCA2 knockdown leads to increased basal plRF3 levels while upon DMXAA stimulation, an increase of pNF-κB protein is observed (Figure 3D). Thus, they show that inhibiting or decreasing SERCA2 protein levels potentiates both acute and chronic STING activation.

Because these data show that SERCA2 is an inhibitor of STING, the inventors next questioned whether SERCA2 activation would increase its inhibitory potential. To this aim, WT-MEFs were treated with the CDN-1163 SERCA2 agonist, and interferon response gene levels were assessed (Figure 4).

The first results show that under DMXAA stimulation, treatment with CDN-1163 inhibits IFN-β gene expression (Figure 4A).

### PERSPECTIVE:

The inventors propose a model where modulation of SERCA2 activity would modify anchorage of STING in the endoplasmic reticulum (ER) membrane (Figure 5). Inhibition of SERCA2 would induce a decrease of Ca²⁺ level in the ER and therefore release STING anchorage by STIM1. STING would be then more capable of being activated and translocated in the Golgi. In contrast, when SERCA2 is activated by its allosteric agonist, an increase in Ca²⁺ level in the ER would fortify STING anchorage by STIM1 and prevent its translocation in the Golgi.

Accordingly, these data demonstrate that SERCA2 inhibitors potentiate the interferon response both in chronic models of STING activation and upon acute stimulations. Based on these data, the invention proposes:
- the use of SERCA2 inhibitors, in the context of cancer or other pathology (not part of the claimed invention) presenting a defective activation of STING and the interferon response. These could include viral pathologies where potentiation of the interferon response could allow faster elimination of the pathogen; and
- the use of SERCA2 agonists to decrease inflammation associated with STING (not part of the claimed invention).
In addition, this could be of potential interest to chronic infectious diseases that include chronic STING activation, as well as to precancerous diseases in chronic inflammatory contexts.

### Example 2: Association of a SERCA2 inhibitor with a STING agonist in cancer cell lines

### MATERIALS AND METHODS

### Cells and cell cultures

Murine embryonic fibroblasts (MEF) and murine pancreatic cancer cell lines (KPC and Panc02) were maintained in Dulbecco's modified Eagle's medium (DMEM) supplemented with 10% fetal bovine serum (FBS), 1% penicillin/streptomycin and 1% glutamine.

### Cell treatment

Cells were treated during 6h with ADUS-100 (5µM) in presence or not of thapsigargin (TA) at 12.5nM or 6.25nM in complete medium.

### RNA extraction and RT-qPCR

RNA was extracted using Trizol (Invitrogen) and treated with TURBO DNase (Ambion) according to manufacturer's protocols. Reverse transcription (SuperScript IV reverse transcriptase, Invitrogen) and qPCR using specific primers were performed using SYBR Green Master Mix (Takara) and LightCycler 480 cycler (Roche). mRNA levels were normalized to HSP90 mRNA levels.

### Viability assay

The CellTiter-Glo^{®} Luminescent Cell Viability Assay was used to determine the number of viable cells 24h after treatment.

### In vivo toxicity

For *in vivo* treatment, mice were shaved under isoflurane anesthesia and injected subcutaneously 3 times with one injection every 3 day. Drugs were solubilized in 5% DMSO/30% PEG-300/1% Tween 80 and 150µl were injected each time at the same site. Three days after the last injection mice were euthanized with CO₂, skin at the injection site was removed and fixed with Formalin 10%.

### RESULTS

Results are shown in Figures 6 and 7.

In order to investigate the impact of SERCA2 inhibition on STING dependent inflammatory responses, several cellular models were used: fibroblastic cell (MEF) and cancer cell lines (KPC and Panc02):
MEF, KPC and Panc02 cells were stimulated or not with ADUS-100, a STING agonist, in combination or not with the thapsigargin SERCA2 inhibitor (TA) (Figure 6A and 7A). Stimulation was performed using 5µM of ADUS-100, a low dose known to poorly trigger IFN responses.

The inventors observed that treatment with SERCA2 inhibitor TA (12.5nM or 6.25nM) in combination with the ADU-S100 STING agonist led to potentiation of induced inflammation markers: Interferon-β (IFN-β) and the CXCL10 cytokine (Figure 6A and 7A, 7C). Treatment with single dose of ADU-S100, thapsigargin or combination were shown to present low cell toxicity (Figure 6B and 7B, 7D).

Mice were injected subcutaneously with (i) NaCl 0.9%, (ii) Vehicle (5% DMSO/30% PEG-300/1% Tween 80), (iii) thapsigargin (TA) at 50nM, ADUS-100 at 5µM, or (iv) TA at 50nM plus ADUS-100 at 5µM (Combi) 3 times (one injection every 3 day). Skin at the injection site were resected. Macroscopic observation did not display signs of toxicity (data not shown).

## Claims

1. Products comprising:
a) at least one inhibitor of SERCA2 (Sarco/Endoplasmic Reticulum Ca²⁺-ATPase 2) selected from the group consisting of thapsigargin, Mipsagargin, JQ-FT, cyclopiazonic acid, artemisinin. CAD204520, Casearin J, CXL017, DBHQ and sHA 14-1, and
b) at least one activator of STING selected from the group consisting of DMXAA, ADU-S100, amidobenzimidazole-based STING agonists, G10, ulevostinag, 3-Amino-bicyclo[3.2.1]octan-8-ol and E7766 ,
as combination products for a simultaneous, separate or sequential use in the treatment and/or prevention of a cancer.

2. Products for use according to claim 1, wherein the inhibitor of SERCA2 is a compound that reversibly or irreversibly binds to SERCA2 and decreases its activity, preferably the inhibitor is non-competitive, preferably the inhibitor is specific of SERCA2.

3. Products for use according to claim 1 or 2, wherein the inhibitor of SERCA2 is thapsigargin or cyclopiazonic acid.

4. Products for use according to any one of claims 1 to 3, wherein the cancer is solid or non-solid, and preferably selected from a colon cancer, a colorectal cancer, a melanoma, a bone cancer, a breast cancer, a thyroid cancer, a prostate cancer, an ovarian cancer, a lung cancer, a pancreatic cancer, a glioma, a cervical cancer, an endometrial cancer, a head and neck cancer, a liver cancer, a bladder cancer, a renal cancer, a skin cancer, a stomach cancer, a testis cancer, an urothelial cancer or an adrenocortical carcinoma, leukemia and lymphoma.

5. Inhibitor of SERCA2 for use in preventing and/or treating a cancer in combination or in association with at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group comprising DMXAA, ADU-S100, amidobenzimidazole-based STING agonists, G10, ulevostinag, 3-Amino-bicyclo[3.2.1]octan-8-ol and E7766.

6. Inhibitor of SERCA2 for use in preventing and/or treating a cancer in a subject treated by at least one activator of STING, wherein the inhibitor of SERCA2 is selected from the group consisting of thapsigargin, Mipsagargin, JQ-FT, cyclopiazonic acid, artemisinin, CAD204520, Casearin J, CXL017, DBHQ and sHA 14-1, and the activator of STING is selected from the group consisting of DMXAA, ADU-S100, amidobenzimidazole-based STING agonists, G10, ulevostinag, 3-Amino-bicyclo[3.2.1]octan-8-ol and E7766.

7. Products for use according to any of claims 1 to 4, or inhibitor for use according to claim 5 or 6, wherein the activator of STING is selected from the group consisting of DMXAA, ADU-S100, amidobenzimidazole-based STING agonists and G10.

## Patentansprüche

1. Produkte, umfassend:
a) mindestens einen Inhibitor von SERCA2 (Sarkoplasma/Endoplasmatisches Retikulum Ca²⁺-ATPase 2), ausgewählt aus der Gruppe bestehend aus Thapsigargin, Mipsagargin, JQ-FT, Cyclopiazonsäure, Artemisinin, CAD204520, Casearin J, CXL017, DBHQ und sHA 14-1, und
b) mindestens einem STING-Aktivator, ausgewählt aus der Gruppe bestehend aus DMXAA, ADU-S100, STING-Agonisten auf Amidobenzimidazol-Basis, G10, Ulevostinag, 3-Amino-bicyclo[3.2.1]octan-8-ol und E7766,
als Kombinationsprodukte zur gleichzeitigen, getrennten oder aufeinanderfolgenden Verwendung bei der Behandlung und/oder Vorbeugung von Krebs.

2. Produkte zur Verwendung gemäß Anspruch 1, wobei der Inhibitor von SERCA2 eine Verbindung ist, die reversibel oder irreversibel an SERCA2 bindet und dessen Aktivität verringert, wobei der Inhibitor vorzugsweise nicht-kompetitiv ist und vorzugsweise spezifisch für SERCA2 ist.

3. Produkte zur Verwendung gemäß Anspruch 1 oder 2, wobei der Inhibitor von SERCA2 Thapsigargin oder Cyclopiazonsäure ist.

4. Produkte zur Verwendung gemäß einem der Ansprüche 1 bis 3, wobei der Krebs solide oder nicht-solide ist und vorzugsweise ausgewählt ist aus einem Dickdarmkrebs, einem kolorektalen Krebs, einem Melanom, einem Knochenkrebs, einem Brustkrebs, einem Schilddrüsenkrebs, einem Prostatakrebs, Eierstockkrebs, Lungenkrebs, Bauchspeicheldrüsenkrebs, Gliom, Gebärmutterhalskrebs, Gebärmutterschleimhautkrebs, Kopf-Hals-Krebs, Leberkrebs, Blasenkrebs, Nierenkrebs, Hautkrebs, Magenkrebs, Hodenkrebs, Urothelkarzinom oder Nebennierenrindenkarzinom, Leukämie und Lymphom.

5. SERCA2-Inhibitor zur Verwendung bei der Vorbeugung und/oder Behandlung einer Krebserkrankung in Kombination oder in Verbindung mit mindestens einem STING-Aktivator, wobei der SERCA2-Inhibitor aus der Gruppe ausgewählt ist, bestehend aus Thapsigargin, Mipsagargin, JQ-FT, Cyclopiazonsäure, Artemisinin, CAD204520, Casearin J, CXL017, DBHQ und sHA 14-1 ausgewählt ist und der STING-Aktivator aus der Gruppe ausgewählt ist, die aus DMXAA, ADU-S100, STING-Agonisten auf Amidobenzimidazol-Basis, G10, Ulevostinag, 3-Amino-bicyclo[3.2.1]octan-8-ol und E7766 besteht.

6. SERCA2-Inhibitor zur Verwendung bei der Vorbeugung und/oder Behandlung einer Krebserkrankung bei einem Patienten, der mit mindestens einem STING-Aktivator behandelt wird, wobei der SERCA2-Inhibitor aus der Gruppe ausgewählt ist, die aus Thapsigargin, Mipsagargin, JQ-FT, Cyclopiazonsäure, Artemisinin, CAD204520, Casearin J, CXL017, DBHQ und sHA 14-1, und der STING-Aktivator aus der Gruppe ausgewählt ist, bestehend aus DMXAA, ADU-S100, STING-Agonisten auf Amidobenzimidazol-Basis, G10, Ulevostinag, 3-Amino-bicyclo[3.2.1]octan-8-ol und E7766.

7. Produkte zur Verwendung gemäß einem der Ansprüche 1 bis 4 oder Inhibitoren zur Verwendung gemäß Anspruch 5 oder 6, wobei der STING-Aktivator aus der Gruppe ausgewählt ist, die aus DMXAA, ADU-S100, STING-Agonisten auf Amidobenzimidazol-Basis und G10 besteht.

## Revendications

1. Produits comprenant :
a) au moins un inhibiteur de SERCA2 (Sarco/Endoplasmic Reticulum Ca²⁺ -ATPase 2) choisi dans le groupe constitué par la thapsigargine, la mipsagargine, le JQ-FT, l'acide cyclopiazonique, l'artémisinine, le CAD204520, la Casearin J, le CXL017, le DBHQ et le sHA 14-1, et
b) au moins un activateur de STING choisi dans le groupe constitué par le DMXAA, l'ADU-S100, les agonistes de STING à base d'amidobenzimidazole, le G10, l'ulevostinag, le 3-amino-bicyclo[3.2.1]octan-8-ol et l'E7766,
en tant que produits combinés destinés à une utilisation simultanée, séparée ou séquentielle dans le traitement et/ou la prévention d'un cancer.

2. Produits pour utilisation selon la revendication 1, dans lesquels l'inhibiteur de SERCA2 est un composé qui se lie de manière réversible ou irréversible à SERCA2 et diminue son activité, de préférence l'inhibiteur est non compétitif, de préférence l'inhibiteur est spécifique de SERCA2.

3. Produits pour utilisation selon la revendication 1 ou 2, dans lesquels l'inhibiteur de SERCA2 est la thapsigargine ou l'acide cyclopiazonique.

4. Produits pour utilisation selon l'une quelconque des revendications 1 à 3, dans lesquels le cancer est solide ou non solide, et de préférence choisi parmi un cancer du côlon, un cancer colorectal, un mélanome, un cancer des os, un cancer du sein, un cancer de la thyroïde, un cancer de la prostate, un cancer de l'ovaire, un cancer du poumon, un cancer du pancréas, un gliome, un cancer du col de l'utérus, un cancer de l'endomètre, un cancer de la tête et du cou, un cancer du foie, un cancer de la vessie, un cancer du rein, un cancer de la peau, un cancer de l'estomac, un cancer des testicules, un cancer urothélial ou un carcinome corticosurrénalien, une leucémie et un lymphome.

5. Inhibiteur de SERCA2 pour utilisation dans la prévention et/ou le traitement d'un cancer en combinaison ou en association avec au moins un activateur de STING, dans lequel l'inhibiteur de SERCA2 est choisi dans le groupe constitué par la thapsigargine, la mipsagargine, le JQ-FT, l'acide cyclopiazonique, l'artémisinine, le CAD204520, la Casearin J, CXL017, le DBHQ et le sHA 14-1, et l'activateur de STING est choisi dans le groupe comprenant le DMXAA, l'ADU-S100, les agonistes de STING à base d'amidobenzimidazole, le G10, l'ulevostinag, le 3-amino-bicyclo[3.2.1]octan-8-ol et l'E7766.

6. Inhibiteur de SERCA2 pour utilisation dans la prévention et/ou le traitement d'un cancer chez un sujet traité par au moins un activateur de STING, dans lequel l'inhibiteur de SERCA2 est choisi dans le groupe constitué par la thapsigargine, la mipsagargine, le JQ-FT, l'acide cyclopiazonique, l'artémisinine, le CAD204520, la Casearin J, CXL017, DBHQ et sHA 14-1, et l'activateur de STING est choisi dans groupe constitué par le DMXAA, de l'ADU-S100, des agonistes de STING à base d'amidobenzimidazole, du G10, de l'ulevostinag, du 3-amino-bicyclo[3.2.1]octan-8-ol et de l'E7766.

7. Produits pour utilisation selon l'une quelconque des revendications 1 à 4, ou inhibiteur pour utilisation selon la revendication 5 ou 6, dans lesquels l'activateur de STING est choisi dans le groupe constitué du DMXAA, de l'ADU-S100, des agonistes de STING à base d'amidobenzimidazole et du G10.
